# EUROPEAN PATENT SPECIFICATION

(11) **EP 3 067 065 B1**
(45) Date of publication and mention of the grant of the patent: **13.05.2020**
(21) Application number: 15158712.8
(22) Date of filing: 11.03.2015
(51) Int. Cl.: A61K 39/35, A61K 39/39, A61K 39/36

(54) **COMPOSITIONS AND TREATMENT OF RESPIRATORY ALLERGIES**
ZUSAMMENSETZUNGEN UND BEHANDLUNG VON ATEMWEGSALLERGIEN
COMPOSITIONS ET TRAITEMENT D'ALLERGIES RESPIRATOIRES

(43) Date of publication of application: 14.09.2016
(73) Proprietor: Anallergo S.r.l., 50038 Scarperia e San Piero (FI) (IT)
(72) Inventor: Ballini, Alessandro, 50032 BORGO SAN LORENZO (FI) (IT); Silvi, Gianna, 50144 FIRENZE (IT)
(74) Representative: Villa, Livia

(56) References cited:
- BR-A- 9 304 388
- GUIMARAES JUNQUEIRA DE QUEIROS M ET AL: "Mite-specific immunotherapy using allergen and/or bacterial extracts in atopic patients in Brazil", JOURNAL OF INVESTIGATIONAL ALLERGOLOGY AND CLINICAL IMMUNOLOGY 2008 ES, vol. 18, no. 2, 2008, pages 84-92, XP55211097, ISSN: 1018-9068
- MAROGNA MAURIZIO: "Effect of Adjuvanted and Standard Sublingual Immunotherapy on Respiratory Function in Pure Rhinitis Due to House Dust Mite over a 5-Year Period", JOURNAL OF ALLERGY AND CLINICAL IMMUNOLOGY, vol. 139, no. 2, Suppl. S, February 2017 (2017-02), page AB62, & ANNUAL MEETING OF THE AMERICAN-ACADEMY-OF-ALLERGY-ASTHMA-AND-IMM UNOLOGY (AAAAI); ATLANTA, GA, USA; MARCH 03 -16, 2017 ISSN: 0091-6749

## Description

### FIELD OF THE INVENTION

The present invention concerns a kit of compositions and a combined anti-allergic therapy for the treatment of respiratory allergies, such as allergic asthma and rhino-conjunctivitis, comprising two sequential phases, i.e. an inductive phase of 2 to 40 weeks, comprising the administration of at least two inactivated bacteria suspensions, to be administered through the nasal/sublingual mucosa, and a specific phase of at least 3 weeks, comprising the administration of at least two mixtures of an allergen-specific extract and an inactivated bacteria suspension, to be administered through the oral/sublingual mucosa.

### STATE OF THE ART

Respiratory allergies such as rhinoconjunctivitis and asthma are due to abnormal immune responses (IgE-mediated sensitization) against otherwise harmless substances called airborne allergens, such as pollens, dust mites, moulds, epithelial derivatives. Allergen-specific immunotherapy is the strategy directed to counteract these altered responses and re-educate the immune system through the administration of the same allergens triggering the disease. The two most used courses of administration adopt the subcutaneous route (injections in the arm) and sublingual route (drops or tablets). In both cases, the therapies protract for at least 3 years, and are usually subdivided in an initial phase (increase of the allergen dose) and a maintenance phase (plateau).

The subcutaneous route is more invasive and not exempt from local and systemic adverse reactions, so that consequently the related administration should be performed in a protected environment and by medical personnel.

The sublingual route, although non-invasive, is characterized by daily or alternate-day dosing regimen, with a very high safety profile and the consequent possibility of administering self-managed by the patient at home.

The allergic immune response is initiated and sustained over time by T helper type 2 (Th2) that stimulate allergen-specific lymphocytes B to the production of IgE antibodies and create the conditions for an allergic reaction of the allergen exposure. This chain of events, namely the induction and activation of lymphocytes type Th2, is triggered by dendritic cells and other "antigen presenting cells" (e.g. macrophages). These cells are deputed to the uptake, processing and presentation to T lymphocytes of antigenic molecules (including allergens) and the polarization of the T lymphocyte response in one of its possible several directions (Th1, Th2, Th17, regulatory T etc.). The Th2 response against the allergens - induced in allergic subjects - is harmful to the body, as it involves precisely the production of IgE antibodies, which cause allergic reactions. The administration of an allergen-specific extract in increasing and high doses aims to achieve the inhibition of the allergic immune response through the stimulation of a T lymphocyte response different from that of Th2, which is able to counteract the effects of IgE antibodies (for example by the induction of blocking antibodies, especially those IgG4 or regulatory T cells) and create the conditions for a so-called clinical and immunological tolerance to the allergen itself. The administration of the current allergen-specific immunotherapy (either subcutaneously or sublingually) requires in any case a latency period before giving results and for this, keeping in mind that the efficiency is dose-dependent, attempts have been done to reduce the latency by significantly increasing the doses administered, thus observing at the same time an undesired increase of the side effects.

In summary, the disadvantages ascribable to the allergen-specific immunotherapy are the following:
- if allergen-specific extracts are used at medium-low doses, the immune response is very slow so that about 3-6 months of therapy are needed to have an acceptable response (e.g. allergies to grass pollen or cypress pollen),
- if allergen-specific extracts are used at high doses, a more rapid immune response (about 3 months) but with serious risks of significant local (and sometimes systemic) side effects,
- the desensitizing allergen-specific immunotherapies do not guarantee an adequate clinical efficacy in polysensitive patients (patients who are allergic to multiple allergens at the same time).

A central role in the (beneficial or harmful) direction of the immune response to allergens is then played by "antigen-presenting cells". The modes and conditions of interaction between these cells and the allergens captured thereby, indeed underlie the type of T lymphocyte response triggered. In general, the physiological condition of these cells is characterized by a continuous operating state, tied to a constant and equally natural exposure to microbial agents. This condition of continued infectious exposure is in fact that normally lived until modern times. In modern times, the hygiene resulted in reduction of microbes' circulation and their diversity, thus causing a reduction in the activation "baseline" of the antigen-presenting cells. Hygiene and new lifestyles is therefore considered the main cause of the so-called "epidemic of allergies".

"Guimaraes et al. ("Mite-Specific Immunotherapy Using Allergen and/or Bacterial Extracts in Atopic Patients in Brazil", J Investig Allergol Clin Immunol 2008; Vol. 18(2): 84-92) concerns a study aimed to evaluate the clinical efficacy and antibody response changes after specific immunotherapy (SIT) using *Dermatophagoides pteronyssinus* (Dpt) allergens with or without bacterial extracts in Brazilian mite-atopic patients. The authors concluded that, when taken together, although there was no clinical efficacy of SIT beyond some placebo effect after 1 year of treatment, and that the serological results showed significant antibody response changes that could be predicting the most favorable SIT group for inducing protection by both reducing IgE synthesis and stimulating the production of IgG blocking antibodies against exacerbation of allergic symptoms in the early period following SIT discontinuation."

In view of the above, an ideal allergen-specific immunotherapy should simultaneously offer an antigenic response (against the allergen, which the patient is sensitive to) and activation of the "innate" component of the immune system (the activation of antigen-presenting cells).

The object of the present invention is to provide an allergen-specific immunotherapy which overcome the drawbacks of the known treatments.

### SUMMARY OF THE INVENTION

The above object has been achieved by a pharmaceutical kit for the treatment of respiratory allergies as claimed in claim 1.

In a further aspect, a pharmaceutical kit for use in an allergen-specific immunotherapy for the treatment of respiratory allergies is disclosed.

The characteristics and the advantages of the present invention will become apparent from the following detailed description and from the working examples provided for illustrative purposes.

### DETAILED DESCRIPTION OF THE INVENTION

The subject of the invention therefore is a pharmaceutical kit comprising:
i) at least two compositions A), each composition A) comprising an inactivated bacteria suspension;
ii) at least two compositions B), each composition B) comprising a mixture of an allergen-specific extract and an inactivated bacteria suspension; and
iii) a leaflet comprising instructions for the sequential use of compositions A) and compositions B) in the treatment of respiratory allergies.

With the term "inactivated", it is meant that the bacteria are grown in culture and then killed using a method such as heat, optionally in the presence of an inactivating chemical agent such as phenol, formaldehyde, thiomersal (also known as thimerosal), or a mixture thereof.

Said inactivated bacteria are selected from those belonging to the following genus: *Klebsiella, Escherichia, Pseudomonas, Staphylococcus, Streptococcus, Haemophilus, Neisseria, Diplococcus, Proteus, Branhamella, Moraxella,* and mixtures thereof.

Preferably, said inactivated bacteria are selected from *Klebsiella pneumoniae, Staphylococcus aureus, Haemophilus influenzae, Streptococcus pneumoniae, Moraxella catharralis, Streptococcus agalactiae, Pseudomonas aeruginosa, Neisseria meningitidis, Streptococcus pyogenes* and combinations thereof.

The preparation of the individual suspensions starts from selected bacterial strains, through specific phases of culture, suspension, titration, inactivation and control. In the subsequent stage, the mixture of inactivated bacteria is prepared by starting from their individual suspensions. The compositions of the kit of the invention are prepared by diluting mixtures of the inactivated bacteria so obtained.

Allergens are molecules capable of eliciting an immune response that is characterized by the presence of specific immunoglobulin E (IgE) antibodies. Most allergens are proteins and all foreign proteins are potential allergens. Allergen-specific extracts are complex mixtures of allergens, enzymes, other proteins, and compounds derived from natural allergen sources. Allergen-specific extracts are prepared by aqueous extraction of allergenic source materials obtained from natural sources.

The preparation of the allergen-specific extract (as per the list herein below) starts from primary raw materials that are processed through controlled stages of extraction, centrifugation, clarification, diafiltration, filtration and control up to a mother extract (active substance). The allergen-specific compositions are obtained by diluting this extract. The allergen-specific extracts of the invention comprise at least one of the relevant respiratory allergens to which an allergic patient is sensitive. In this regard, said allergen is selected from those listed below:

### 1. Tree pollens

*Betula verrucosa* = *B. pendula* (correct taxonomic name according to NCBI taxonomic database) = *B. alba* European white birch
*Alnus glutinosa* Alder
*Carpinus betulus* Hornbeam
*Corylus avellana* Hazel
*Quercus alba* Oak
*Olea europaea* Olive
*Fraxinus excelsior* Ash
*Ligustrum vulgare* Privet
*Syringa vulgaris* Lilac
*Juniperus sp.* Cedar
*Cupressus sp.* Cypress
*Fagus sylvatica* European beech
*Castanea sativa* Sweet Chestnut or Marron
*Acer sp.* Maple
*Platanus sp.* Plane tree
*Populus sp.* Poplar
*Robinia pseudoacacia* False acacia, Locust tree
*Salix sp. Sallow* / *Willow*
*Tilia sp. Linden* / Lime tree
*Ulmus sp.* Elm
*Oyptomeria japonica* Japanese Cedar

### 2. Grass and cereal pollens

*Anthoxanthum odoratum* Sweet vernal grass
*Avena sativa* Oat
*Dactylis glomerata* Orchard grass/Cocksfoot
*Festuca sp. Meadow fescue*
*Holcus lanatus* Velvet grass/Yorkshire fog
*Hordeum vulgare* Barley
*Lolium perenne* Perennial ryegrass
*Phleum pratense* Timothy grass
*Poa pratensis* Kentucky bluegrass
*Secale cereale* Cultivated rye
*Triticum aestivum* Cultivated wheat
*Agropyron sp.* Couch grass, Crested wheatgrass
*Agrostis sp.* Bent grass
*Alopecurus pratensis* Meadow foxtail
*Arrhenatherum elatius* False oat
*Bromus sp.* Brome grass
*Cynodon dactylon* Bermuda grass
*Cynosurus cristatus* Dogstail

### 3. Wild herb pollens

*Ambrosia artemisiifolia, Ambrosia trifida* Ragweed
*Artemisia vulgaris* Mugwort
*Parietaria judaica, Parietaria officinalis Pellitory*
*Plantago sp.* Plantain

### 4. Mites

*Dermatophagoides pteronyssinus*
*Dermatophagoides farinae*
*Euroglyphus maynei*
*Avarus siro* flour mite
*Glycyphagus domesticus* house mite
*Lepidoglyphus destructor* house mite
*Thyreophagus entomophagus* flour mite
*Tyrophagus putrescentiae* storage mite

### 6. Epithelial derivatives (hair, dandruff)

*Canis familiaris* Dog
*Felis domesticus* Cat
*Cavia porcellus* Guinea pig
*Cricetus cricetus* Hamster
*Equus caballus* Horse
*Mus musculus* Mouse
*Oryctolagus cuniculus* Rabbit
*Rattus sp.* Rat

### 7. Moulds

*Alternaria alternata (tenuis)*
*Aspergillus fumigatus*
*Cladosporium herbarum*

### 8. Others

*Blattella germanica*
Peanuts

In some embodiments, the allergen-specific extracts of the invention comprise two different respiratory allergens to which an allergic patient is sensitive, preferably as follows:
- *Graminacee* mix (Grass mix) and *Parietaria* spp (Pellitory)
- *Graminacee* mix (Grass mix) and *Betula* (Birch)
- *Graminacee* mix (Grass mix) and *Asteraceae* (Ragweed, Mugworth, Taraxacum)
- *Graminacee* mix (Grass mix) and *Olea* (Olive)
- *Graminacee* mix (Grass mix) and *Cupressus* (Cypress)
- *Asteraceae* (Ragweed, Mugworth, Taraxacum) and *Parietaria* spp (Pellitory)
- *Olea* (Olive) and *Parietaria* spp (Pellitory)
- *Betula* (Birch) and *Cupressus* (Cypress)
- *Parietaria* spp (Pellitory) and *Cupressus* (Cypress)
- Dust mites mix (*D.Pter, D.far., E.may*) and Epitelium *Felis domesticus* (Cat)
- *Betula* (Birch) - *Corylus* (Hazel)

The leaflet comprises instructions for the sequential use of compositions A) and compositions B) in the treatment of respiratory allergies. This means that compositions B) are instructed to be administered after compositions A). More precisely, it is instructed that each composition is to be individually administered following this sequential order: a first composition A) is to be administered as a first stage, and subsequently a second composition A) is to be administered as a second stage, and so on for all the compositions A); the same is then instructed for the compositions B).

It has been surprisingly found that, in the treatment of respiratory allergies, the initial administration of compositions A) comprising inactivated bacteria suspensions in a first inductive phase triggers the activation of the innate component of the immune system (i.e. the activation of the antigen-presenting cells). The subsequent administration of compositions B) introduces the allergen in an already activated immune system and continues to stimulate the immune system with the inactivated bacteria.

The main advantage of the invention is therefore the boosting of the therapy efficacy, by conveniently decreasing the concentration of the allergen-specific extract, which implies the reduction of the possible side effects. The reduction of side effects advantageously results in an improved compliance to therapy.

Preferably, each composition A) comprises an inactivated bacteria suspension having a title of 2 to 400 million inactivated bacteria/ml, and each composition B) comprises a mixture of an allergen-specific extract having a title of 100 to 20'000 RAST Units/ml and an inactivated bacteria suspension having a title of 200 to 400 million inactivated bacteria/ml.

'RAST Units/ml' correspond to the protein concentration in µg/ml, which is constant and defined for each allergen-specific extract for each composition B). This protein concentration is detected by an *in vitro* total allergenic activity test, so that 10'000 RAST Units/ml correspond to the inhibition of at least 50% of the response of a pool of sera containing IgE specific to the allergen.

Preferably, the inactivated bacteria suspensions in said at least two compositions A) have different titles from one another.

Preferably, the inactivated bacteria suspensions in said at least two compositions B) have different titles from one another.

Preferably, the mixture of an allergen-specific extracts in said at least two compositions B) have different titles from one another.

In preferred embodiments, the leaflet comprises instructions for the sequential use of compositions A) and compositions B), wherein said at least two compositions A) are instructed to be administered starting from the composition A) having the lowest title to the composition A) having the highest title, and said at least two compositions B) are instructed to be administered starting from the composition B) having the lowest title to the composition B) having the highest title.

More preferably, each composition A) comprises an inactivated bacteria suspension having a title of 10 to 350 million inactivated bacteria/ml, and each composition B) comprises a mixture of an allergen-specific extract having a title of 300 to 15'000 RAST Units/ml and an inactivated bacteria suspension having a title of 250 to 350 million inactivated bacteria/ml.

The inactivated bacteria are selected from the group consisting of *K. pneumoniae, H. influenzae, S. pneumoniae, S. aureus,* and combinations thereof.

In other preferred embodiments, the kit of the invention comprise three to five compositions A).

In further preferred embodiments, the kit of the invention comprise three to five compositions B).

More preferred embodiments comprise four compositions A) and four compositions B). The most preferred embodiments comprise:
i) four compositions A) as follows
   - A1) containing 10-15 million inactivated bacteria/ml;
   - A2) containing 30-35 million inactivated bacteria/ml;
   - A3) containing 80-120 million inactivated bacteria/ml;
   - A4) containing 280-320 million inactivated bacteria/ml;
      or as follows
   - A1) containing 30-35 million inactivated bacteria/ml;
   - A2) to A4), each containing 280-320 million inactivated bacteria/ml;
   and
ii) four compositions B) as follows
   - B1) containing an allergen-specific extract 350-450 RAST Units/ml, and an inactivated bacteria suspension containing 280-320 million of inactivated bacteria/ml;
   - B2) to B4), each containing an allergen-specific extract 9'000-11'000 RAST Units/ml, and an inactivated bacteria suspension containing 280-320 million inactivated bacteria/ml;
   wherein all the inactivated bacteria suspensions comprise *Klebsiella pneumoniae, Staphylococcus aureus, Haemophilus influenzae* and *Streptococcus pneumoniae.*

Said compositions further comprise suitable pharmaceutically acceptable excipients such as pH adjusters, osmotic agents, emulsifiers, dispersing agents, surfactants, solubilizers, buffering agents, diluents, preservatives, wetting agents, gelling agents, consistency agents, chelating agents, ciliary stimulants, mucus thinning agents, suspending agents, thickening agents, or combinations thereof.

Particularly, the pH adjuster can be sodium hydroxide, orthophosphoric acid, citric acid, sodium citrate, sodium hydrogen sulphate, borate buffer, sodium hydrogen orthophosphate, disodium hydrogen phosphate, sodium dihydrogen phosphate, trometamol, acetate buffer, citrate buffer or mixtures thereof.

The osmotic agent can be sodium chloride, potassium chloride, zinc chloride, calcium chloride, mannitol, glycerol, and boric acid, citric acid, sodium tartrate, sodium phosphate, potassium phosphate, propylene glycol or other inorganic or organic solutes, dextrose, anhydrous glucose or mixtures thereof.

The surfactant can be an amphoteric, non-ionic, cationic or anionic surfactant or combinations thereof.

The preservative can be phenol, benzalkonium chloride, benzoic acid, sodium benzoate, potassium sorbate, sorbic acid, edetic acid or its alkali salts, lower alkyl p-hydroxybenzoates, chlorhexidine, phenyl mercury borate, quaternary ammonium compound or mixtures thereof.

The consistency agents can be monosaccharides, disaccharides and other sugars, ribose, glycerine/glycerol, gelatin (fish source), sorbitol, xylitol, inositol, propylene glycol, galactose, mannose, xylose, rhamnose, glutaraldehyde, invert sugars, ethanol, honey, mannitol, threalose, polyethylene glycol or mixtures thereof.

The chelating agent can be sodium EDTA or disodium EDTA or mixtures thereof.

The diluents can be sodium hydrogen carbonate, calcium hydrogen phosphate dihydrate, sodium monohydrogen phosphate dihydrate or mixtures thereof.

Preferred excipients are sodium chloride, glycerin, phenol, threalose, gelatin (fish source), mannitol, sodium hydrogen carbonate, calcium hydrogen phosphate dihydrate, sodium monohydrogen phosphate dihydrate, potassium phosphate monobasic, sodium hydroxide, orthophosphoric acid and mixtures thereof.

The most preferred excipients are sodium chloride, glycerin, phenol, sodium hydroxide, orthophosphoric acid, and mixtures thereof.

The compositions of the kit can be in the form of a tablet, capsule, mini-tablet, micro-tablet, granule, microgranule, pellet, multiparticulate, micronized particulate, spray, solution, suspension, ointment, gel, or drops. Preferably, the compositions of the kit can be in the form of a spray or drops.

In a further aspect, the present invention concerns the pharmaceutical kit above described, for use in an allergen-specific immunotherapy for the treatment of respiratory allergies. Said respiratory allergies include allergic asthma, allergic rhinitis, allergic conjunctivitis, and allergic rhino-conjunctivitis.

The inactivated bacteria suspensions are able to play a crucial role in the activation of the immune system. The innate immune response is activated whenever the body is exposed to potentially pathogenic agents (for example, some bacterial species). It is exactly the presence of inactivated bacteria (in particular if they consist of whole and potentially pathogenic cells), which puts the allergen-specific extract in the conditions to be better treated and processed by antigen-presenting cells, physiologically activated and efficient and therefore able to induce a protective immune response. It has been found that a very successful therapy for the treatment of patients suffering from mild or moderate allergic rhinoconjunctivitis and allergic asthma, is the therapy of the present invention that includes a first inductive phase of stimulation of the immune system with a mixture of inactivated bacteria suspensions followed by a stage with a mixture of allergen-specific extracts and inactivated bacteria suspensions. This therapy can be also proposed for polysensitive patients (allergic to multiple allergens).

The use of the pharmaceutical kit comprises two sequential phases, i.e. an inductive phase of 2 to 40 weeks, comprising the administration of at least two compositions A) to be administered through the nasal/sublingual mucosa, and a specific phase of at least 3 weeks, comprising the administration of at least two compositions B) to be administered through the oral/sublingual mucosa.

Particularly, said at least two compositions A) are to be administered before said at least two compositions B), wherein the route of administration is oral, sublingual, buccal, or nasal.

Preferably, the administration of said at least two compositions A) is carried out over a period of time of 2 to 40 weeks, and the administration of said at least two compositions B) is carried out over a period of time of 2 to 50 weeks.

More preferably, the administration of said at least two compositions A) is carried out over a period of time of 5 to 20 weeks, and the administration of said at least two compositions B) is carried out over a period of time of 3 to 40 weeks.

In some preferred embodiments, each composition A) is to be administered over a period of time of 1 to 10 weeks.

In other preferred embodiments, each composition B) is to be administered over a period of time of 1 to 10 weeks.

Preferably said at least two compositions A) are to be administered starting from the composition A) having the lowest title to the composition A) having the highest title, and said at least two compositions B) are to be administered starting from the composition B) having the lowest title to the composition B) having the highest title.

More preferably the composition A) having the lowest title is to be administered for a period of time of 1 to 3 weeks, and the composition A) having the highest title is to be administered for a period of time of 2 to 6 weeks.

And also more preferably, the composition B) having the lowest title is to be administered for a period of time of 1 to 3 weeks, and the composition B) having the highest title is to be administered for a period of time of 5 to 9 weeks.

In the most preferred embodiments, the compositions of the kit are in the form of spray or drops, and the kit comprises:
i) four compositions A) as follows
   - A1) containing 10-15 million inactivated bacteria/ml, to be administered for 1-2 weeks;
   - A2) containing 30-35 million inactivated bacteria/ml, to be administered for 1-4 weeks;
   - A3) containing 80-120 million inactivated bacteria/ml, to be administered for 3-5 weeks;
   - A4) containing 280-320 million inactivated bacteria/ml, to be administered for 3-5 weeks;
      or as follows
   - A1) containing 30-35 million inactivated bacteria/ml, to be administered for 1-2 weeks;
   - A2) to A4), each containing 280-320 million inactivated bacteria/ml, each to be administered for 3-5 weeks;
   and
ii) four compositions B) as follows
   - B1) containing an allergen-specific extract 350-450 RAST Units/ml, and an inactivated bacteria suspension containing 280-320 million of inactivated bacteria/ml, to be administered for 1-2 weeks;
   - B2) to B4), each containing an allergen-specific extract 9'000-11'000 RAST Units/ml, and an inactivated bacteria suspension containing 280-320 million inactivated bacteria/ml, each to be administered for 6-8 weeks.

Within said most preferred embodiments, the compositions B1) even more preferably comprise an allergen-specific extract 400 RAST Units/ml, as follows in Table 1:

**Table 1.**

| Allergen | RAST Units/ml | Total protein concentration (µg/ml) Sublingual 4 ml | Dilution with 1 ml of suspension of inactivated bacteria 300 million/ml | Peak dose (µg/ml) | Peak dose (µg) (16 drops) |
|---|---|---|---|---|---|
| | | | | 16 drops of 0.045 ml = 0.72 ml | |
| Wheat flour | 400 | 0.2 | 0.16 | 0.72 | 0.12 |
| Epitelium *Canis familiaris* | 400 | 0.4 | 0.32 | 0.72 | 0.23 |
| *Olea europea Corylus avellana, Ciraminacee* mix (*Phleum, Poa, Cynodon*) *Parietaria spp* ,Salsola kali | 400 | 1.8 | 1.44 | 0.72 | 1.04 |
| *Cupressus arizonica, Betula alba Asteracee* mix (*Taraxacum officinalis, Ambrosia elatior, Artemisia vulgaris) Alternaria tenuis* | 400 | 1.2 | 0.96 | 0.72 | 0.69 |
| Moulds Mix (*Alternaria tenuis* 1/3; *Aspergillus spp* 1/3; *Cladosporium* 1/3) *Alternaria* 30 µg; *Aspergillus* 60 µg; *Cladosporium* 10 µg | 400 | 1.33 | 1.06 | 0.72 | 0.76 |
| Epitelium of *Felis domesticus* | 400 | 2.6 | 2.08 | 0.72 | 1.50 |
| Dust Mites mix (*Dermatophagoides pteronyssinus, Dermatophagoides farinae, Euroglyphus maynei*), *Dermatophagoides pteronyssinus, Dermatophagoides farinae,* Dust Mites mix (*Dermatophagoides pteronyssinus, Dermatophagoides farinae*) | 400 | 4 | 3.2 | 0.72 | 2.30 |

Within said most preferred embodiments, the compositions B2) even more preferably comprise an allergen-specific extract 10'000 RAST Units/ml, as follows in Table 2:

**Table 2.**

| Allergen | RAST Units/ml | Total protein concentration (µg/ml) Sublingual 4 ml | Dilution with 1 ml of suspension of inactivated bacteria 300 million/ml | Peak dose (µg/ml) | Peak dose (µg) (5 drops) |
|---|---|---|---|---|---|
| | | | | 5 drops of 0.045ml = 0.225ml | |
| Wheat flour | 10'000 | 5 | 4 | 0.225 | 0.90 |
| Epitelium *Canis familiaris* | 10'000 | 10 | 8 | 0.225 | 1.80 |
| *Olea europea Corylus avellana Graminacee mix (Phleum, Poa, Cynodon) Parietaria spp Salsola kali* | 10'000 | 45 | 36 | 0.225 | 8.10 |
| *Cupressus arizonica Betula alba Asteracee* mix (*Taraxacum officinalis, Ambrosia elatior, Artemisia vulgaris) Alternaria tenuis* | 10'000 | 30 | 24 | 0.225 | 5.40 |
| Moulds Mix (*Alternaria tenuis* 1/3; *Aspergillus spp* 1/3; *Cladosporium* 1/3) *Alternaria 30* µg; *Aspergillus* 60 µg; *Cladosporium* 10 µg | 10'000 | 33.3 | 26.64 | 0.225 | 5.99 |
| Epitelium of *Felis domesticus* | 10'000 | 65 | 52 | 0.225 | 11.70 |
| Dust Mites mix (*Dermatophagoides pteronyssinus, Dermatophagoides farinae, Euroglyphus maynei*) *Dermatophagoides pteronyssinus Dermatophagoides farinae* Dust Mites mix (*Dermatophagoides pteronyssinus, Dermatophagoides farinae*) | 10'000 | 100 | 80 | 0.225 | 18.00 |

It should be appreciated that the kit of the invention allows to significantly reduce the amounts of the allergen dose to be administered, owing to the activation of the immune system obtained by the administration of inactivated bacteria suspensions as composition A) and as components of the compositions B). In this regard, it should be also considered that commercial products containing allergens, as available to date, are instructed to be administered at remarkably higher doses of allergens, such as peak doses up to 200 µg, where conversely the inventive compositions B) can be administered at very low doses as reported in the tables above.

When the compositions of the kit are in the form of a spray, said compositions are administered in an amount of 1-2 puffs/day/nostril.

When the compositions of the kit are in the form of drops, said compositions are administered in an amount of 1-10 drops/day.

When the compositions of the kit are in the form of a solid unit such as a tablet, capsule, mini-tablet, or micro-tablet, said compositions are administered in an amount of 1-5 solid units/day.

It should be understood that all aspects identified as preferred and advantageous for the pharmaceutical kit are to be deemed as similarly preferred and advantageous also for the respective processes of production and use in treating respiratory allergies of the present invention.

It should be also understood that all the combinations of preferred aspects of the pharmaceutical kit, their processes of production, as well as their uses in treating respiratory allergies, as above reported, are to be deemed as hereby disclosed.

Below are working examples of the present invention provided for illustrative purposes.

### EXAMPLES

### Example 1.

### Mixture of inactivated bacteria

The preparation of the individual suspensions starts from selected bacterial strains, through specific phases of culture, suspension, titration, inactivation and control. In the subsequent stage is prepared the mixture of inactivated bacteria starting from the individual suspensions.

The components of the mixture of inactivated bacteria (titrated to 3-6 billion germs/ml) in a phenol-saline solution are the following:
- inactivated bacterial suspension of *K. pneumoniae* (25%)
- inactivated bacterial suspension of *H. influenzae* (25%)
- inactivated bacterial suspension of *S. pneumoniae* (25%)
- inactivated bacterial suspension of *S. aureus* (25%).

The compositions used in the combined anti-allergic therapy of the invention are prepared by diluting mixtures of the inactivated bacteria described above.

### Allergen-specific extract

The preparation of the allergen-specific extract starts from primary raw materials that are processed through controlled stages of extraction, centrifugation, clarification, diafiltration, filtration on 0.2 µm and control up to a mother extract (active substance). The allergen-specific compositions are obtained by diluting this extract.

### Example 2.

### KIT OF COMPOSITIONS FOR THE COMBINED ANTI-ALLERGIC THERAPY COMPRISING A) TOPICAL NASAL COMPOSITIONS FOR THE INDUCTIVE PHASE AND B) SUBLINGUAL COMPOSITIONS FOR THE SPECIFIC PHASE

### A) Topical nasal compositions for the inductive phase

The compositions are suspensions comprising inactivated bacteria of four strains (i.e. *Klebsiella pneumoniae, Staphylococcus aureus, Haemophilus influenzae* and *Streptococcus pneumoniae*), sodium chloride, glycerin, phenol and water for injections. The compositions optionally can contain also sodium hydroxide and orthophosphoric acid for pH adjustment.

Specifically, four compositions have been prepared having different inactivated bacteria concentrations, as follows:
- bottle 1 (orange label) contains 11 million inactivated bacteria/ml;
- bottle 2 (orange label) contains 33 million inactivated bacteria/ml;
- bottle 3 (orange label) contains 100 million inactivated bacteria/ml;
- bottle 4 (orange label) contains 300 million inactivated bacteria/ml.

### B) Sublingual compositions for the specific phase

The compositions are suspensions comprising an allergen-specific extract and inactivated bacteria of four strains (i.e. *Klebsiella pneumoniae, Staphylococcus aureus, Haemophilus influenzae* and *Streptococcus pneumoniae*), sodium chloride, glycerin, phenol and water for injections. The compositions optionally can contain also sodium hydroxide and orthophosphoric acid for pH adjustment.

Specifically, four compositions have been prepared having different allergen-specific extract and inactivated bacteria concentrations, as follows:
- 1 bottle (green label) contains 4 ml of an allergen-specific extract 400 RAST Units/ml, and 1 ml of an inactivated bacteria mixture containing 300 million of inactivated bacteria/ml;
- 3 bottles (red label) containing 4 ml of an allergen-specific extract 10'000 RAST Units/ml, and 1 ml of an inactivated bacteria mixture containing 300 million inactivated bacteria/ml.

Depending on the allergen selected, the allergen-specific extract 400 RAST Units/ml of bottle (green label) has the following features:

**Table 3.**

| Allergen | RAST Units/ml | Total protein concentration (µg/ml) Sublingual 4 ml | Dilution with 1 ml of suspension of inactivated bacteria 300 million/ml | Peak dose (µg/ml) | Peak dose (µg) (16 drops) |
|---|---|---|---|---|---|
| | | | | 16 drops of 0.045 ml = 0.72 ml | |
| Wheat flour | 400 | 0.2 | 0.16 | 0.72 | 0.12 |
| Epitelium *Canis familiaris* | 400 | 0.4 | 0.32 | 0.72 | 0.23 |
| *Olea europea Corylus avellana Graminacee mix (Phleum, Poa, Cynodon) Parietaria spp Salsola kali* | 400 | 1.8 | 1.44 | 0.72 | 1.04 |
| *Cupressus arizonica Betula alba Asteracee* mix (*Taraxacum officinalis, Ambrosia elatior, Artemisia vulgaris) Alternaria tenuis* | 400 | 1.2 | 0.96 | 0.72 | 0.69 |
| Moulds Mix (*Alternaria tenuis* 1/3; *Aspergillus spp* 1/3; *Cladosporium* 1/3) *Alternaria* 30 µg; *Aspergillus* 60 µg; *Cladosporium* 10 µg | 400 | 1.33 | 1.06 | 0.72 | 0.76 |
| Epitelium of *Felis domesticus* | 400 | 2.6 | 2.08 | 0.72 | 1.50 |
| Dust Mites mix (*Dermatophagoides pleronyssinus, Dermatophagoides farinae, Euroglyphus maynei*) *Dermatophagoides pleronyssinus Dermatophagoides farinae* Dust Mites mix (*Dermatophagoides pleronyssinus, Dermatophagoides farinae*) | 400 | 4 | 3.2 | 0.72 | 2.30 |

and the allergen-specific extract 10'000 RAST Units/ml of bottles (red label) has the following features:

**Table 4.**

| Allergen | RAST Units/ml | Total protein concentration (µg/ml) Sublingual 4 ml | Dilution with 1 ml of suspension of inactivated bacteria 300 million/ml | Peak dose (µg/ml) | Peak dose (µg) (5 drops) |
|---|---|---|---|---|---|
| | | | | 5 drops of 0.045ml = 0.225ml | |
| Wheat flour | 10'000 | 5 | 4 | 0.225 | 0.90 |
| Epitelium *Canis familiaris* | 10'000 | 10 | 8 | 0.225 | 1.80 |
| *Olea europea Corylus avellana Graminacee mix (Phleum, Poa, Cynodon) Parietaria spp Salsola kali* | 10'000 | 45 | 36 | 0.225 | 8.10 |
| *Cupressus arizonica Betula alba Asteracee* mix (*Taraxacum officinalis, Ambrosia elatior, Artemisia vulgaris) Alternaria tenuis* | 10'000 | 30 | 24 | 0.225 | 5.40 |
| Moulds Mix (*Alternaria tenuis* 1/3; *Aspergillus spp* 1/3; *Cladosporium* 1/3) *Alternaria* 30 µg; *Aspergillus* 60 µg; *Cladosporium* 10 µg | 10'000 | 33.3 | 26.64 | 0.225 | 5.99 |
| Epitelium of *Felis domesticus* | 10'000 | 65 | 52 | 0.225 | 11.70 |
| Dust Mites mix (*Dermatophagoides pleronyssinus, Dermatophagoides farinae, Euroglyphus maynei*) *Dermatophagoides pteronyssinus Dermatophagoides farinae* Dust Mites mix (*Dermatophagoides pleronyssinus, Dermatophagoides farinae*) | 10'000 | 100 | 80 | 0.225 | 18.00 |

### Administration scheme

In the Table 5, the scheme of administration is reported:

**Table 5.**

| Bottle | n. of weeks | amount |
|---|---|---|
| 1 orange | 1 | 1 puff/day/nostril |
| 2 orange | 1 | 1 puff/day/nostril |
| 3 orange | 4 | 1 puff/day/nostril |
| 4 orange | 4 | 1 puff/day/nostril |
| 5 green | 1,1 | from 2 drops/day (day 1) to 16 drops/day (day 8) |
| 6 red | 7 | 1-5 drops/day |
| 7 red | 7 | 1-5 drops/day |
| 8 red | 7 | 1-5 drops/day |
| Total | 32,1 (7,5 months) | |

### Example 3.

### KIT OF COMPOSITIONS FOR THE COMBINED ANTI-ALLERGIC THERAPY COMPRISING A) SUBLINGUAL COMPOSITIONS FOR THE INDUCTIVE PHASE AND B) SUBLINGUAL COMPOSITIONS FOR THE SPECIFIC PHASE

### A) Sublingual compositions for the inductive phase

The compositions are suspensions comprising inactivated bacteria of four strains (i.e. *Klebsiella pneumoniae, Staphylococcus aureus, Haemophilus influenzae* and *Streptococcus pneumoniae*), sodium chloride, glycerin, phenol and water for injections. The compositions optionally can contain also sodium hydroxide and orthophosphoric acid for pH adjustment.

Specifically, four compositions have been prepared having different inactivated bacteria concentrations, as follows:
- 1 bottle (green label) contains 30 million inactivated bacteria/ml;
- 3 bottles (red label) contains 300 million inactivated bacteria/ml.

### B) Sublingual compositions for the specific phase

The compositions are suspensions comprising an allergen extract and inactivated bacteria of four strains (i.e. *Klebsiella pneumoniae, Staphylococcus aureus, Haemophilus influenzae* and *Streptococcus pneumoniae*), sodium chloride, glycerin, phenol and water for injections. The compositions optionally can contain also sodium hydroxide and orthophosphoric acid for pH adjustment.

Specifically, four compositions have been prepared having different allergen-specific extract and inactivated bacteria concentrations, as follows:
- 1 bottle (green label) contains 4 ml of an allergen-specific extract 400 RAST Units/ml, and 1 ml of an inactivated bacteria mixture containing 300 million of inactivated bacteria/ml;
- 3 bottles (red label) containing 4 ml of an allergen-specific extract 10'000 RAST Units/ml, and 1 ml of an inactivated bacteria mixture containing 300 million inactivated bacteria/ml.

Depending on the allergen selected, the allergen-specific extract 400 RAST Units/ml of bottle (green label) and the allergen-specific extract 10'000 RAST Units/ml of bottle (green label) have the same features as reported in Example 2.

### Administration scheme

In the Table 6, the scheme of administration is reported:

**Table 6.**

| Bottle | n. of weeks | amount |
|---|---|---|
| 1 green | 1.4 | 1-5 drops/day |
| 2 red | 4 | 1-5 drops/day |
| 3 red | 4 | 1-5 drops/day |
| 4 red | 4 | 1-5 drops/day |
| 5 green | 1.1 | from 2drops/day (day 1) to 16 drops/day(day 8) |
| 6 red | 7 | 1-5 drops/day |
| 7 red | 7 | 1-5 drops/day |
| 8 red | 7 | 1-5 drops/day |
| Total | 35.5 (8.2 months) | |

### Example 4.

### KIT OF COMPOSITIONS FOR THE COMBINED ANTI-ALLERGIC THERAPY COMPRISING A) TOPICAL NASAL COMPOSITIONS FOR THE INDUCTIVE PHASE AND B) SUBLINGUAL COMPOSITIONS FOR THE SPECIFIC PHASE

The Example 2 has been repeated, with the difference that the allergen-specific extract comprises one of the following combinations of two different respiratory allergens:
- *Graminacee* mix (Grass mix) and *Parietaria spp* (Pellitory)
- *Graminacee* mix (Grass mix) and *Betula* (Birch)
- *Graminacee* mix (Grass mix) and *Asteraceae* (Ragweed, Mugworth, Taraxacum)
- *Graminacee* mix (Grass mix) and *Olea* (Olive)
- *Graminacee* mix (Grass mix) and *Cupressus* (Cypress)
- *Asteraceae* (Ragweed, Mugworth, Taraxacum) and Parietaria spp (Pellitory)
- *Olea* (Olive) and *Parietaria spp* (Pellitory)
- *Betula* (Birch) and *Cupressus* (Cypress)
- *Parietaria spp* (Pellitory) and *Cupressus* (Cypress)
- Dust mites mix (*D.Pter, D.far., E.may*) and Epitelium *Felis domesticus* (Cat)
- *Betula* (Birch) - *Corylus* (Hazel)

### Example 5.

### KIT OF COMPOSITIONS FOR THE COMBINED ANTI-ALLERGIC THERAPY COMPRISING A) SUBLINGUAL COMPOSITIONS FOR THE INDUCTIVE PHASE AND B) SUBLINGUAL COMPOSITIONS FOR THE SPECIFIC PHASE

The Example 3 has been repeated, with the difference that the allergen-specific extract comprises one of the following combinations of two different respiratory allergens:
- *Graminacee* mix (Grass mix) and *Parietaria spp* (Pellitory)
- *Graminacee* mix (Grass mix) and *Betula* (Birch)
- *Graminacee* mix (Grass mix) and *Asteraceae* (Ragweed, Mugworth, Taraxacum)
- *Graminacee* mix (Grass mix) and *Olea* (Olive)
- *Graminacee* mix (Grass mix) and *Cupressus* (Cypress)
- *Asteraceae* (Ragweed, Mugworth, Taraxacum) and *Parietaria spp* (Pellitory)
- *Olea* (Olive) and *Parietaria spp* (Pellitory)
- *Betula* (Birch) and *Cupressus* (Cypress)
- *Parietaria spp* (Pellitory) and *Cupressus* (Cypress)
- Dust mites mix (*D.Pter, D.far., E.may*) and Epitelium *Felis domesticus* (Cat)
- *Betula* (Birch) - *Corylus* (Hazel)

## Claims

1. A pharmaceutical kit for the treatment of respiratory allergies comprising:
i) at least two compositions A) to be administered through the nasal or sublingual mucosa, each composition A) comprising an inactivated bacteria suspension;
ii) at least two compositions B) to be administered through the oral or sublingual mucosa, each composition B) comprising a mixture of an allergen-specific extract and an inactivated bacteria suspension; and
iii) a leaflet comprising instructions for the sequential use of compositions A) and compositions B) in the treatment of respiratory allergies;
wherein said inactivated bacteria suspension comprises a combination of *K. pneumoniae, H. influenzae, S. pneumoniae,* and *S. aureus,*
wherein each composition A) comprises an inactivated bacteria suspension having a titer of 10 to 350 million inactivated bacteria/ml, and each composition B) comprises a mixture of an allergen-specific extract having a titer of 300 to 15'000 RAST Units/ml and an inactivated bacteria suspension having a titer of 250 to 350 million inactivated bacteria/ml,
wherein allergen is an airborne allergen, and
wherein sequential use means that said at least two compositions A) are to be administered before said at least two compositions B).

2. The pharmaceutical kit of claim 1, wherein the compositions of the kit are in the form of a spray or drops.

3. The pharmaceutical kit of claim 1 or 2, wherein the inactivated bacteria suspensions in said at least two compositions A) have different titers from one another, the inactivated bacteria suspensions in said at least two compositions B) have different titers from one another, the mixture of an allergen-specific extracts in said at least two compositions B) have different titers from one another.

4. The pharmaceutical kit of any one of claims 1-3, comprising three to five compositions A) and three to five compositions B).

5. The pharmaceutical kit of claim 4, comprising four compositions A) and four compositions B).

6. The pharmaceutical kit of any one of claims 1-5, comprising:
i) four compositions A) as follows
- A1) containing 10-15 million inactivated bacteria/ml;
- A2) containing 30-35 million inactivated bacteria/ml;
- A3) containing 80-120 million inactivated bacteria/ml;
- A4) containing 280-320 million inactivated bacteria/ml;
or as follows
- A1) containing 30-35 million inactivated bacteria/ml;
- A2) to A4), each containing 280-320 million inactivated bacteria/ml;
and
ii) four compositions B) as follows
- B1) containing an allergen-specific extract 350-450 RAST Units/ml, and an inactivated bacteria suspension containing 280-320 million of inactivated bacteria/ml;
- B2) to B4), each containing an allergen-specific extract 9'000-11'000 RAST Units/ml, and an inactivated bacteria suspension containing 280-320 million inactivated bacteria/ml;
wherein all the inactivated bacteria suspensions comprise *Klebsiella pneumoniae, Staphylococcus aureus, Haemophilus influenzae* and *Streptococcus pneumoniae.*

7. The pharmaceutical kit of claim 1 for use in an allergen-specific immunotherapy for the treatment of respiratory allergies, wherein said at least two compositions A) are to be administered starting from the composition A) having the lowest titer to the composition A) having the highest titer, and said at least two compositions B) are to be administered starting from the composition B) having the lowest titer to the composition B) having the highest titer.

8. The pharmaceutical kit for use of claim 7, wherein the administration of said at least two compositions A) is carried out over a period of time of 2 to 40 weeks, and the administration of said at least two compositions B) is carried out over a period of time of 2 to 50 weeks.

9. The pharmaceutical kit for use of claim 8, wherein the administration of said at least two compositions A) is carried out over a period of time of 5 to 20 weeks, and the administration of said at least two compositions B) is carried out over a period of time of 3 to 40 weeks.

10. The pharmaceutical kit for use of claim 7, wherein each composition A) is to be administered over a period of time of 1 to 10 weeks.

11. The pharmaceutical kit for use of any one of claims 7 or 10, wherein, each composition B) is to be administered over a period of time of 1 to 10 weeks.

12. The pharmaceutical kit for use of any one of claims 7, 10 or 11, wherein the composition A) having the lowest titer is to be administered for a period of time of 1 to 3 weeks, and the composition A) having the highest titer is to be administered for a period of time of 2 to 6 weeks; and wherein the composition B) having the lowest titer is to be administered for a period of time of 1 to 3 weeks, and the composition B) having the highest titer is to be administered for a period of time of 5 to 9 weeks.

13. The pharmaceutical kit for use of claim 12, wherein the compositions of the kit are in the form of spray or drops, and the kit comprises:
i) four compositions A) as follows
- A1) containing 10-15 million inactivated bacteria/ml, to be administered for 1-2 weeks;
- A2) containing 30-35 million inactivated bacteria/ml, to be administered for 1-4 weeks;
- A3) containing 80-120 million inactivated bacteria/ml, to be administered for 3-5 weeks;
- A4) containing 280-320 million inactivated bacteria/ml, to be administered for 3-5 weeks;
or as follows
- A1) containing 30-35 million inactivated bacteria/ml, to be administered for 1-2 weeks;
- A2) to A4), each containing 280-320 million inactivated bacteria/ml, each to be administered for 3-5 weeks;
and
ii) four compositions B) as follows
- B1) containing an allergen-specific extract 350-450 RAST Units/ml, and an inactivated bacteria suspension containing 280-320 million of inactivated bacteria/ml, to be administered for 1-2 weeks;
- B2) to B4), each containing an allergen-specific extract 9'000-11'000 RAST Units/ml, and an inactivated bacteria suspension containing 280-320 million inactivated bacteria/ml, each to be administered for 6-8 weeks.

14. The pharmaceutical kit for use of any one of claim 7-13, wherein when the compositions of the kit are in the form of a spray, said compositions are administered in an amount of 1-2 puffs/day/nostril.

15. The pharmaceutical kit for use of any one of claim 7-13, wherein when the compositions of the kit are in the form of drops, said compositions are administered in an amount of 1-10 drops/day.

## Patentansprüche

1. Pharmazeutisches Kit für die Behandlung von Atemwegsallergien, bestehend aus:
i) mindestens zwei Zusammensetzungen A) zur Verabreichung durch die Nasenschleimhaut oder die sublinguale Schleimhaut, wobei jede Zusammensetzung A) eine inaktivierte Bakteriensuspension umfasst;
ii) mindestens zwei Zusammensetzungen B) zur Verabreichung durch die orale oder sublinguale Schleimhaut, wobei jede Zusammensetzung B) eine Mischung aus einem allergenspezifischen Extrakt und einer inaktivierten Bakteriensuspension umfasst; und
iii) eine Packungsbeilage, die Anleitungen für die aufeinanderfolgende Anwendung der Zusammensetzungen A) und B) bei der Behandlung von Atemwegsallergien enthält;
wobei die inaktivierte Bakteriensuspension eine Kombination von *K. pneumoniae, H. influenzae, S. pneumoniae* und *S. aureus* umfasst,
wobei jede Zusammensetzung A) eine inaktivierte Bakteriensuspension mit einem Titer von 10 bis 350 Millionen inaktivierten Bakterien/ml umfasst und jede Zusammensetzung B) eine Mischung aus einem allergenspezifischen Extrakt mit einem Titer von 300 bis 15.000 RAST-Einheiten/ml und einer inaktivierten Bakteriensuspension mit einem Titer von 250 bis 350 Millionen inaktivierten Bakterien/ml umfasst,
wobei das Allergen ein durch die Luft übertragenes Allergen ist, und
wobei die aufeinanderfolgende Anwendung bedeutet, dass die mindestens zwei Zusammensetzungen A) vor den mindestens zwei Zusammensetzungen B) zu verabreichen sind.

2. Pharmazeutisches Kit gemäß Anspruch 1, wobei die Zusammensetzungen des Kits in Form eines Sprays oder von Tropfen vorliegen.

3. Pharmazeutisches Kit gemäß Anspruch 1 oder 2, wobei die inaktivierten Bakteriensuspensionen in den mindestens zwei Zusammensetzungen A) unterschiedliche Titer voneinander haben, die inaktivierten Bakteriensuspensionen in den mindestens zwei Zusammensetzungen B) unterschiedliche Titer voneinander haben, die Mischung eines allergenspezifischen Extrakts in den mindestens zwei Zusammensetzungen B) unterschiedliche Titer voneinander haben.

4. Pharmazeutisches Kit gemäß einem der Ansprüche 1 - 3, umfassend drei bis fünf Zusammensetzungen A) und drei bis fünf Zusammensetzungen B).

5. Pharmazeutisches Kit gemäß Anspruch 4, bestehend aus vier Zusammensetzungen A) und vier Zusammensetzungen B).

6. Pharmazeutisches Kit gemäß einem der Ansprüche 1 - 5, umfassend:
i) vier Zusammensetzungen A) wie folgt
- A1) enthält 10 - 15 Millionen inaktivierte Bakterien/ml;
- A2) enthält 30 - 35 Millionen inaktivierte Bakterien/ml;
- A3) enthält 80 - 120 Millionen inaktivierte Bakterien/ml;
- A4) enthält 280 - 320 Millionen inaktivierte Bakterien/ml; oder wie folgt
- A1) enthält 30 - 35 Millionen inaktivierte Bakterien/ml;
- A2) bis A4), die jeweils 280 - 320 Millionen inaktivierte Bakterien/ml enthalten;
und
ii) vier Kompositionen B) wie folgt
- B1) enthält einen allergenspezifischen Extrakt mit 350 - 450 RAST-Einheiten/ml und eine inaktivierte Bakteriensuspension mit 280 - 320 Millionen inaktivierten Bakterien/ml;
- B2) bis B4), die jeweils einen allergenspezifischen Extrakt mit 9.000-11.000 RAST-Einheiten/ml und eine inaktivierte Bakteriensuspension mit 280 - 320 Millionen inaktivierten Bakterien/ml enthalten;
wobei alle inaktivierten Bakteriensuspensionen *Klebsiella pneumoniae, Staphylococcus aureus, Haemophilus influenzae* und *Streptococcus pneumoniae* umfassen.

7. Pharmazeutisches Kit gemäß Anspruch 1 zur Anwendung in einer allergenspezifischen Immuntherapie zur Behandlung von Atemwegsallergien, wobei die mindestens zwei Zusammensetzungen A) ausgehend von der Zusammensetzung A) mit dem niedrigsten Titer zu der Zusammensetzung A) mit dem höchsten Titer zu verabreichen sind, und die mindestens zwei Zusammensetzungen B) ausgehend von der Zusammensetzung B) mit dem niedrigsten Titer zu der Zusammensetzung B) mit dem höchsten Titer zu verabreichen sind.

8. Pharmazeutisches Kit zur Anwendung gemäß Anspruch 7, wobei die Verabreichung der mindestens zwei Zusammensetzungen A) über einen Zeitraum von 2 bis 40 Wochen und die Verabreichung der mindestens zwei Zusammensetzungen B) über einen Zeitraum von 2 bis 50 Wochen durchgeführt wird.

9. Pharmazeutisches Kit zur Anwendung gemäß Anspruch 8, wobei die Verabreichung der mindestens zwei Zusammensetzungen A) über einen Zeitraum von 5 bis 20 Wochen und die Verabreichung der mindestens zwei Zusammensetzungen B) über einen Zeitraum von 3 bis 40 Wochen durchgeführt wird.

10. Pharmazeutisches Kit zur Anwendung gemäß Anspruch 7,wobei jede Zusammensetzung A) über einen Zeitraum von 1 bis 10 Wochen zu verabreichen ist.

11. Pharmazeutisches Kit zur Anwendung gemäß einem der Ansprüche 7 oder 10, wobei jede Zusammensetzung B) über einen Zeitraum von 1 bis 10 Wochen zu verabreichen ist.

12. Pharmazeutisches Kit zur Anwendung gemäß einem der Ansprüche 7, 10 oder 11, wobei die Zusammensetzung A) mit dem niedrigsten Titer über einen Zeitraum von 1 bis 3 Wochen und die Zusammensetzung A) mit dem höchsten Titer über einen Zeitraum von 2 bis 6 Wochen zu verabreichen ist, und wobei die Zusammensetzung B) mit dem niedrigsten Titer über einen Zeitraum von 1 bis 3 Wochen und die Zusammensetzung B) mit dem höchsten Titer über einen Zeitraum von 5 bis 9 Wochen zu verabreichen ist.

13. Pharmazeutisches Kit zur Anwendung gemäß Anspruch 12, wobei die Zusammensetzungen des Kits in Form von Spray oder Tropfen vorliegen und das Kit umfasst:
i) vier Zusammensetzungen A) wie folgt
- A1) enthält 10 - 15 Millionen inaktivierte Bakterien/ml, die für 1 - 2 Wochen zu verabreichen sind;
- A2) enthält 30 - 35 Millionen inaktivierte Bakterien/ml, die für 1 - 4 Wochen zu verabreichen sind;
- A3) enthält 80 - 120 Millionen inaktivierte Bakterien/ml, die für 3 - 5 Wochen zu verabreichen sind;
- A4) enthält 280 - 320 Millionen inaktivierte Bakterien/ml, die für 3 - 5 Wochen zu verabreichen sind;
oder wie folgt
- A1) enthält 30 - 35 Millionen inaktivierte Bakterien/ml, die für 1 - 2 Wochen zu verabreichen sind;
- A2) bis A4), die jeweils 280 - 320 Millionen inaktivierte Bakterien/ml enthalten, die jeweils für 3 - 5 Wochen zu verabreichen sind;
und
ii) vier Zusammensetzungen B) wie folgt
- B1) enthalten einen allergenspezifischen Extrakt 350 - 450 RAST-Einheiten/ml und eine inaktivierte Bakteriensuspension mit 280 - 320 Millionen inaktivierten Bakterien/ml, die für 1 - 2 Wochen zu verabreichen sind;
- B2) bis B4), die jeweils einen allergenspezifischen Extrakt 9.000 - 11.000 RAST-Einheiten/ml und eine inaktivierte Bakteriensuspension mit 280 - 320 Millionen inaktivierten Bakterien/ml enthalten, die jeweils für 6 - 8 Wochen zu verabreichen sind.

14. Pharmazeutisches Kit zur Anwendung gemäß einem der Ansprüche 7 - 13, wobei, wenn die Zusammensetzungen des Kits in Form eines Sprays vorliegen, diese Zusammensetzungen in einer Menge von 1 - 2 Zügen/Tag/Nasenloch verabreicht werden.

15. Pharmazeutisches Kit zur Anwendung gemäß einem der Ansprüche 7 - 13, wobei, wenn die Zusammensetzungen des Kits in Form von Tropfen vorliegen, diese Zusammensetzungen in einer Menge von 1 - 10 Tropfen/Tag verabreicht werden.

## Revendications

1. Kit pharmaceutique pour le traitement des allergies respiratoires comprenant :
i) au moins deux compositions A) à administrer à travers la muqueuse nasale ou sublinguale, chaque composition A) comprenant une suspension de bactéries inactivées ;
ii) au moins deux compositions B) à administrer à travers la muqueuse orale ou sublinguale, chaque composition B) comprenant un mélange d'un extrait spécifique d'allergène et d'une suspension de bactéries inactivées ; et
iii) une notice comprenant des instructions pour l'utilisation séquentielle des compositions A) et des compositions B) dans le traitement des allergies respiratoires ;
dans lequel ladite suspension de bactéries inactivées comprend une combinaison de *K. pneurnoniae,*
*H. injluenzae, S. pneumoniae et S. aureus,*
dans lequel chaque composition A) comprend une suspension de bactéries inactivées ayant un titre de 10 à 350 millions de bactéries inactivées/ml, et chaque composition B) comprend un mélange d'un extrait spécifique des allergènes ayant un titre de 300 à 15 000 unités RAST/ml et une suspension de bactéries inactivées ayant un titre de 250 à 350 millions de bactéries inactivées/ml,
dans lequel l'allergène est un allergène aéroporté, et
dans lequel l'utilisation séquentielle signifie que lesdites au moins deux compositions A) doivent être administrées avant lesdites au moins deux compositions B).

2. Kit pharmaceutique selon la revendication 1, dans lequel les compositions du kit se présentent sous forme
de spray ou de gouttes.

3. Kit pharmaceutique selon la revendication 1 ou 2, dans lequel les suspensions de bactéries inactivées dans lesdites au moins deux compositions A) ont des titres différents les unes des autres, les suspensions de bactéries inactivées dans lesdites au moins deux compositions B) ont des titres différents les unes des autres, le mélange d'extraits spécifiques des allergènes dans lesdites au moins deux compositions B) ont des titres différents les unes des autres.

4. Kit pharmaceutique selon l'une quelconque des revendications 1 à 3, comprenant trois à cinq compositions A) et trois à cinq compositions B).

5. Kit pharmaceutique selon la revendication 4, comprenant quatre compositions A) et quatre compositions B).

6. Kit pharmaceutique selon l'une quelconque des revendications 1 à -5, comprenant :
i) quatre compositions A) comme suit
- A1) contenant 10-15 millions de bactéries inactivées/ml ;
- A2) contenant 30-35 millions de bactéries inactivées/ml ;
- A3) contenant 80-120 millions de bactéries inactivées/ml ;
- A1) contenant 280-320 millions de bactéries inactivées/ml ; ou comme suit
- A1) contenant 30-35 millions de bactéries inactivées/ml ;
- A2) à A4), contenant chacune 280-320 millions de bactéries inactivées/ml ;
et
ii) quatre compositions A) comme suit
- B1) contenant un extrait spécifique des allergènes 350-450 RAST Units/ml, et une suspension de bactéries inactivées contenant 280-320 millions de bactéries inactivées/ml ;
- B2) à B4), contenant chacune un extrait spécifique des allergènes 9000-11 000 unités RAST/ml, et une suspension de bactéries inactivées contenant 280-320 millions de bactéries inactivées/ml ;
dans lequel toutes les suspensions de bactéries inactivées constituent *Klebsiella pneumoniae, Staphylococcus aureus, Haemophilus influenzae* et *Streptococcus pneumonia.*

7. Kit pharmaceutique selon la revendication 1, pour l'utilisation dans une immunothérapie spécifique des allergènes pour le traitement des allergies respiratoires, dans lequel lesdites au moins deux compositions A) doivent être administrées en partant de la composition A) ayant le titre le plus bas vers la composition A) ayant le titre le plus élevé, et lesdites au moins deux compositions B) doivent être administrées en partant de la composition B) ayant le titre le plus bas vers la composition B) ayant le titre le plus élevé.

8. Kit pharmaceutique pour l'utilisation selon la revendication 7, dans lequel l'administration desdites au moins deux compositions A) est effectuée sur une période de temps de 2 à 40 semaines, et l'administration desdites au moins deux compositions B) est effectuée sur une période de temps de 2 à 50 semaines.

9. Kit pharmaceutique pour l'utilisation selon la revendication 8, dans lequel l'administration desdites au moins deux compositions A) est effectuée sur une période de temps de 5 à 20 semaines, et l'administration desdites au moins deux compositions B) est effectuée sur une période de temps de 3 à 40 semaines.

10. Kit pharmaceutique pour l'utilisation selon la revendication 7
dans lequel chaque composition A)
doit être administrée sur une période de 1 à 10 semaines.

11. Kit pharmaceutique pour l'utilisation selon l'une quelconque des revendications 7 ou 10 dans lequel chaque composition
B) doit être administrée sur une période de 1 à 10 semaines.

12. Kit pharmaceutique pour l'utilisation selon l'une quelconque des revendications 7, 10 ou 11 dans lequel chaque composition A)
ayant le titre le plus bas doit être administrée pendant une période de temps de 1 à 3 semaines, et la composition A) ayant le titre le plus élevé doit être administrée pendant une période de temps de 2 à 6 semaines ; et dans lequel la composition B) ayant le titre le plus bas doit être administrée pendant une période de temps de 1 à 3 semaines, et la composition B) ayant le titre le plus élevé doit être administrée pendant une période de temps de 5 à 9 semaines.

13. Kit pharmaceutique pour l'utilisation selon la revendication 12, dans lequel les compositions du kit sont sous forme de spray ou de gouttes, et le kit comprend :
i) quatre compositions A) comme suit
- A1) contenant 10-15 millions de bactéries inactivées/ml, à administrer pendant 1 ou 2 semaines ;
- A2) contenant 30-35 millions de bactéries inactivées/ml, à administrer pendant 1 à 4 semaines;
- A3) contenant 80-120 millions de bactéries inactivées/ml, à administrer pendant 3 à 5 semaines ;
- A4) contenant 280-320 millions de bactéries inactivées/ml, à administrer pendant 3 à 5 semaines ;
ou comme suit
- A1) contenant 30-35 millions de bactéries inactivées/ml, à administrer pendant 1 ou 2 semaines ;
- A2) à A4), contenant chacune 280-320 millions de bactéries inactivées/ml, à administrer pendant 3 a 5 semaines ;
ii) quatre compositions A) comme suit
- B1) contenant un extrait spécifique des allergènes 350-450 Unités RAST/ml, et une suspension de bactéries inactivées contenant 280-320 millions de bactéries inactivées/ml, à administrer pendant 1 à 2 semaines ;
- B2) à B4), contenant chacune un extrait spécifique des allergènes 9000-11 000 unités RAST/ml, et une suspension de bactéries inactivées contenant 280-320 millions de bactéries inactivées/ml, a administré pendant 6 à 8 semaines ;

14. Kit pharmaceutique pour l'utilisation selon l'une quelconque des revendications 7 à 13, dans lequel, lorsque les compositions du kit sont sous forme de spray, lesdites compositions sont administrées en une quantité de 1 à 2 bouffées/jour/narine

15. Kit pharmaceutique pour l'utilisation selon l'une quelconque des revendications 7 à 13, dans lequel, lorsque les compositions du kit sont sous forme de gouttes, lesdites compositions sont administrées en une quantité de 1 à 10 gouttes/jour.
